# NEW EUROPEAN PATENT SPECIFICATION

(11) **EP 0 547 482 B2**
(45) Date of publication and mention of the opposition decision: **30.08.2006**
(45) Mention of the grant of the patent: 06.05.1998
(21) Application number: 92120924.3
(22) Date of filing: 09.12.1992
(51) Int. Cl.: A61N 1/30

(54) **Iontophoresis system having features for reducing skin irritation**
Mit Mittel zur Verminderung der Hautreizung ausgerüstetes iontophoretisches System
Système d'ionophorese comportant des moyens de réduction de l'irritation de la peau

(30) Priority: 17.12.1991 US 808754; 10.03.1992 US 848217
(43) Date of publication of application: 23.06.1993
(73) Proprietor: Vyteris, Inc., Fair Lawn, New Jersey 07410-2700 (US)
(72) Inventor: Haynes, John L., Chapell Hill, NC 27514 (US); Sage, Burton H. Jr., Raleigh, NC 27613 (US)
(74) Representative: Lawrence, Peter Robin Broughton

(56) References cited:
- EP-A- 0 277 314
- WO-A-91/09645
- FR-A- 1 603 649
- US-A- 4 109 645
- US-A- 4 141 359
- US-A- 4 292 968
- US-H-71 (MEDTRONIC)
- US Statutory Inventio Registratio H516.

## Description

This invention relates to a system and method for iontophoretic drug delivery having features for reducing irritation to the skin of an animal and more particularly, to a system for delivery of power during iontophoretic drug delivery in a sequence for reducing skin irritation.

Iontophoresis is gaining increased acceptance as an effective method for application of ionic agents or ionic drugs through the skin of an animal. Iontophoresis can be defined as the electrically driven application of drugs or medications, in their ionic form, to the surface tissues of an animal. The application of electric current causes migration of ions into the tissue wherein such migration is proportional to the quantity of current applied through the iontophoretic system.

Skin irritation can occur during iontophoretic drug delivery. Efforts to minimize irritation have been directed to regulating the level of current, improving the electrical connection of the electrode with the skin and reducing the hydrolysis of water in the ionic medication, Irritation of the skin may be subjective wherein the iontophoretic electrode delivers so much power that it causes extreme discomfort to the patient There are also objective indicia of irritation such as petechia, erythemia and edema. Occurrence of such forms of irritation is discussed by Nancy A. Monteiro-Riviere in a paper presented in Fundamental Applied Technology entitled "Altered Epidermal Morphology Secondary to Lidocaine lontophoresis: In Vivo and In Vitro Studies in Porcaine Skin", Vol 15, pages 174-185 (1990).

It is known that the impedance of a patient's skin can range from over 100,000 ohms to nearly 1000 ohms, depending on the duration that the iontophoretic current is applied, the magnitude of the current which is being delivered, the location of the system on the patient's body, and other factors. In a system where the desired current level, which is determined in part by the drug administered to the patient, is one milliamp, a voltage potential of 100 volts would result if the skin impedance is 100,000 ohms. Since such a voltage would cause undesirable sensations to the user, it is highly desirable to limit the voltage across the electrodes of the iontophoretic drug delivery device to a more tolerable level.

Numerous prior art references attempt to teach iontophoretic devices which attempt to avoid irritation and/or tissue damage. US-A-4,292,968 discloses an apparatus for delivering constant current during ion therapy wherein the therapy is provided by silver ions released from a silver anode. The current will abruptly switch to delivering constant voltage when the voltage across the electrodes reaches a predetermined level. US-A-4,725,263 teaches a programmable constant current source transdermal drug delivery system wherein the current level can be adjusted or preset by trimming a circuit board in the apparatus.

However, the dual mode power source described in the US-A-4 292 968 is impractical for use with a transdermal drug delivery system. The power source disclosed in US-A-4 292 968 employs a voltage limit of 1.1 volts. While skin impedance levels in man can range as low as. 1 ,000 ohms, more typical values during drug delivery are in the range of 5,000-10,000 ohms. This leaves a current typically in the range of 100 to 200 µA, a level which will fail to deliver much drug.

Also, the dual mode power source first disdosed in US-A-4 292 968 provides a constant current to the electrodes of the drug delivery device, and then switches to a constant voltage if the voltage across the electrodes exceeds 1.1 volts. As described with respect to the present invention, the opposite sequence, that is; changing from a constant voltage to constant current, takes place and is used in reducing skin irritation and burning.

US-A-4,141,359 teaches an epidermal iontophoresis device which is capable of maintaining a constant current through the epidermal tissue. To prevent excessive voltage build-up and the accompanying dangers of shock and burns, a comparative circuit monitors current flow and voltage across the electrodes and automatically triggers and SCR shut down circuit when impedance readings are outside of predetermined limits.

US-A-4 141 359 thus describes an iontophoresis power source which is a constant current source with an output voltage capable of reaching 60 volts. There is no means to prevent the voltage from reaching this level which can cause adverse sensations to the patient. Described in this patent is a safety mechanism which is activated if the patch is removed while power is being delivered. This mechanism checks the impedance of the load and turns the system off if there is a large sudden change in the impedance.

As mentioned above, it is well known that undesirable sensations will arise when voltages of this level (60 volts) are applied to the skin. To avoid these sensations, the circuit described in US-A-4 141 359 provides for the user to set the level of controlled current, and in this way avoid the sensations.

US-A-4,211,222 teaches an iontophoretic burn protection method. US-A-4 211 222 teaches the use of an electrically conductive porous intervener having a thickness which is large in relation to the thickness of the skin. This intervener is interposed between a first electrode and the skin. US-A-4 211 222 also teaches that pain and tingling due to the passage of electric current can be reduced by increasing the area of the electrode delivering the drug. US-A-4,164,226 teaches iontophoretic burn-protection electrode structures wherein one electrode of an iontophoretic system has a porous material of a thickness in excess of 3 millimeters interposed between the electrode and the skin.

US-A-4,764,164 teaches an iontophoresis device which includes an electric source including a pulse generator. The device has a circuit for discharging the charges accumulated in the electrodes during each intermission period of therapeutic pulses generated by the pulse generator. US-A-4,764,164 teaches that the device can be easily applied to the human skin, without causing undesirable irritation in the skin, and especially without causing burns and rubefaction in the skin. WO 91/09645 describes a pulsed delivery system wherein the pulses have dual-segment coaveform characteristics which are selected to provide efficient administration without increasing skin irritation.

Although the prior art is replete with devices for reducing skin irritation and skin damage, the prior art efforts appear to focus on the device itself. Devices running on lower voltages, devices having intermediate pads between the electrodes and the skin, devices producing pulsating current, and devices having large electrode areas to reduce current concentration all approach the problem from a device perspective. The prior art has not attempted to understand the resistivity of the skin and develop a system that can take advantage of the natural properties of the skin in order to optimize the iontophoretic delivery while minimizing irritation and skin damages.

It is the object of the present invention to provide an improved device for delivering a drug iontophoretically to an animal as well as an electrical control circuit included in such an iontophoretic drug delivery system.

This object is solved, according to the invention, with the features of claims 1

It is an advantage of the present invention to provide a drug delivery device which is attachable to a patient for supplying drug to the patient by iontophoresis, and an electronic circuit which operates with the drug delivery device to control the amount of drug and the rate at which the drug is delivered.

It is a further advantage of the present invention to provide an iontophoretic drug delivery system which minimizes or eliminates undesirable irritation, burning and rubefaction of the skin of the animal to which the system is attached.

It is yet another advantage of the present invention to provide an electronic circuit which selectively provides a controlled voltage and controlled current to the electrodes of an iontophoretic drug delivery device attachable to a patient.

It is still another advantage of the present invention to provide an iontophoresis system having an electronic circuit which monitors the current and voltage provided to the electrodes of a drug delivery device attachable to the skin of a patient for selectively controlling the amount or rate of current or voltage applied to the electrodes of the drug delivery device.

It is still a further advantage of the present invention to provide an iontophoretic drug delivery system which overcomes the inherent drawbacks of known systems.

In accordance with the present invention, the iontophoretic drug delivery system includes a device having a drug reservoir adapted to be placed in communication with the skin of an animal and an electrolyte reservoir which is adapted to be placed in communication with the skin of the animal. The device includes two electrodes. The first electrods is mounted in the drug reservoir, and the second electrode is mounted in the electrolyte reservoir.

According to a further preferred embodiment of the drug delivery system includes an electronic circuit coupled to the electrodes of the device having the drug reservoir and the electrolyte reservoir, which circuit selectively provides at least one of a controlled voltage and a controlled current to the electrodes. The circuit includes an adjustable voltage and current generating circuit coupled to the electrodes, a current sensor coupled to one or more of the electrodes for sensing the current provided to the electrodes, and a voltage sensor coupled to one or more of the electrodes for sensing the voltage provided to the electrodes. The current and voltage sensors respectively generate a sensed current feedback signal and a sensed voltage feedback signal, which are representative of the electrode current and voltage sensed by the sensors.

The electronic circuit further includes a comparator circuit for comparing the sensed current feedback signal with a predetermined current threshold signal. A feedback signal selector circuit which is responsive to at least an output signal from the current comparator circuit will generate a feedback signal corresponding to at least one of the sensed current feedback signal and the sensed voltage feedback signal.

The electronic circuit further includes a setpoint selector circuit which is responsive to at least the output signal from the current comparator circuit and, in response thereto, will generate a setpoint signal corresponding to at least one of a voltage limit signal and a desired delivery current signal.

A subtractor circuit will subtract the feedback signal from the setpoint signal and generate an error signal corresponding to the difference between the two. The voltage and current generating circuit is responsive to this error signal and adjusts at least one of the current and voltage provided to the electrodes of the drug delivery device.

These and other features and advantages of this invention will become apparent from the following detailed description of illustrative embodiments thereof, which is to be read in connection with the accompanying drawings, in which:
Fig. 1 is a perspective view of an iontophoretic drug delivery system of the present invention;
Fig. 2 is a partial cross-sectional view of the drug delivery device of Fig. 1 taken along line 2-2;
Fig. 3 and Fig. 4 are graphs iiiustrating voltage, current and power;
Fig. 5 is a schematic/block diagram view showing a first embodiment of a circuit for providing current for the iontophoretic delivery of the present invention:
Fig. 5A is a block diagram of a second embodiment of the electronic circuit of the iontophoresis system formed in accordance with the present invention;
Fig. 6 is a graph illustrating a profile of current with respect to time for iontophoretic delivery of the present invention; and
Fig. 7 is a graph illustrating Voltage with respect to time for iontophoretic delivery using the present invention.

White this invention is satisfied by embodiments in many different forms, there are shown in the drawings and will herein be described in detail preferred embodiments of the invention with the understanding that the present disclosure is to be considered exemplary of the principles of the invention.

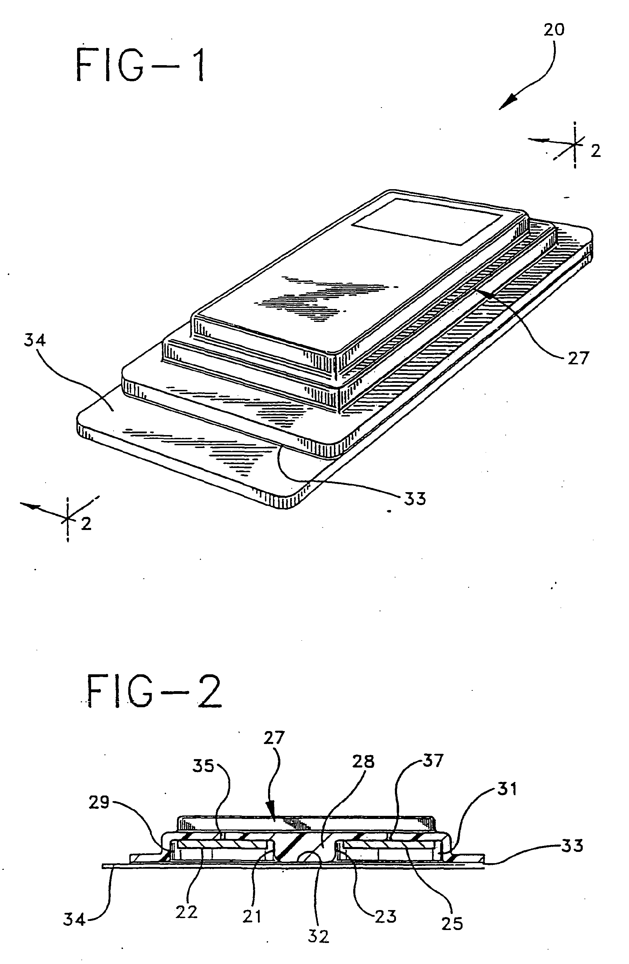

Adverting to Figs. 1-7 an operable iontaphoretic drug delivery system/device 20 includes a drug reservoir 21 adapted to be attached to the skin of an animal, a first electrode 22 in the drug reservoir, an electrolyte reservoir 23 adapted to be attached to the skin of an animal and a second electrode 25 in the electrolyte reservoir.

For the purposes of the description of the present invention, the term "proximal" or "lower" is meant to refer to the side of the device closest to the skin, whereas the term "distal" or "upper" is meant to refer to the side of the device or ' element which is furthest from the skin.

For the purpose of description of the present invention and the claims, the term "animal" as used herein shall include all living beings including humans The term "irritation" as used herein shall mean subjective irritation such as pain and tingling and objective irritation such as petechia, erythemia and edema.

In this embodiment, the entire housing is made of insulating material, such as plastic.

Drug reservoir 21 is capable of holding an ionic compound such as a therapeutic compound, a diagnostic compound and a drug. In many cases, the ionic compounds are ionic liquids, however, the compound may be in the form of a gel or may be contained in the reservoir along with other materials such as porous polymeric structures. For the purpose of the description of this invention, drug reservoir 21 contains a therapeutic liquid 29. This therapeutic liquid does not limit the invention but is intended to be representative of these many possibilities for an ionic compound which can be delivered iontophoretically.

Electrolyte reservoir 23 contains electrolyte solution 31. The electrolyte solution may be in the form of a liquid or a gel, or may be contained in the reservoir along with other materials such as porous polymeric material.

Lower surface 32 of the housing contains an adhesive coating 33 for attaching the housing to the skin of an animal. A removable release sheet 34 is provided to protect the adhesive before time of use and for helping to contain the therapeutic liquid and the electrolyte in the reservoirs before time of use.

The drug delivery device shown in Figs. 1 and 2 further includes conductors 35 and 37 which are respectively connected to electrodes 22 and 25 and to the electronic circuit of the iontophoresis system of the present invention. The conductors may be made of silver, sliver/chloride, gold, copper, molybdenum, lead or other suitable conductive materials.

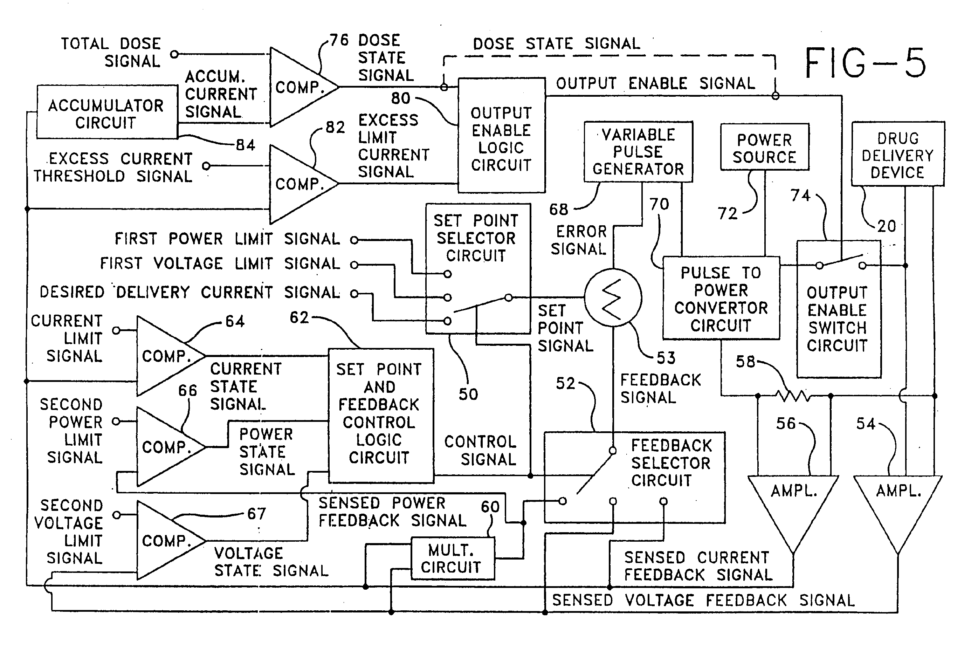

A substantial difference between the iontophoretic drug delivery device of the present invention and the prior art lies in the electronic circuitry, one form of which is shown in Fig. 5. To fully understand the major structural and functional differences between the device of the present invention and the prior art it is important to understand the properties of the skin into which ions are driven by an iontophoretic drug delivery device. The electrical impedance of the skin greatly affects the occurrence of irritation and tissue damage. The electrical impedance of epidermal tissue is highly variable, depending on such factors as location on the body, presence of calluses or dermal abrasions, ambient air conditions such as temperature and humidity, state of hydration which may be caused by perspiration, and the age, sex and race of the individual. It is known that the dose of an ionic substance delivered to an individual is in general proportional to the electric current. In efforts to increase the dose of ionic substance or reduce the time over which a given dose may be administered, the electric current is often raised to a level which results in skin irritation and skin damage including burns. For example, in US-A-4,141,359 native values of skin impedance of 10,000 to 50,000 ohms are disclosed. If a modest current of 3 milliamperes is caused to flow through skin with an average value of 30,000 ohms, the required voltage by Ohms' Law would be 30,000 ohms time 0.003 amperes or 90 volts. Such a voltage is widely known to be capable of causing irreversible skin breakdown, which is believed to provide low resistance paths, and hence paths where high levels of energy are dissipated in the skin. This high level of energy is believed to cause burns.

During iontophoresis episodes, the impedance of skin through which ions are transferred is initially at a high value, and then steadily decreases. The instant invention provides a device and a method for effective iontophoretic delivery while minimizing irritation and burns by accommodating the highly variable nature of skin impedance.

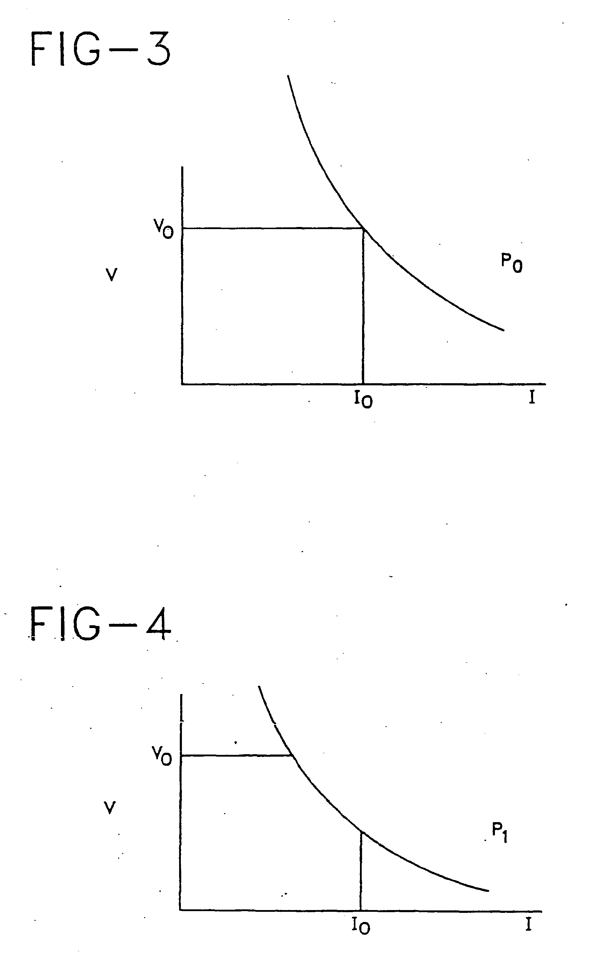

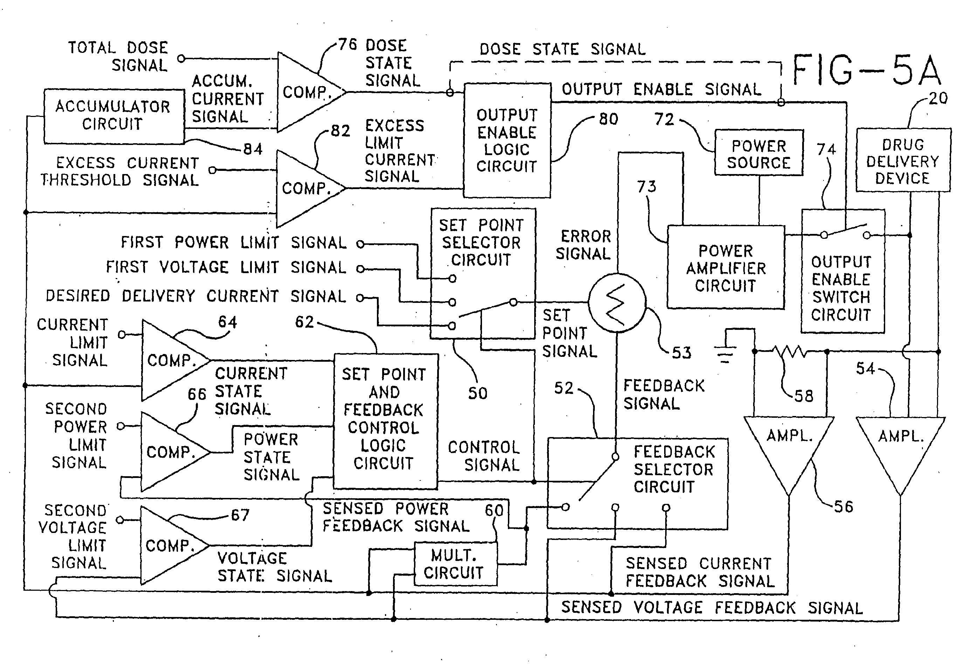

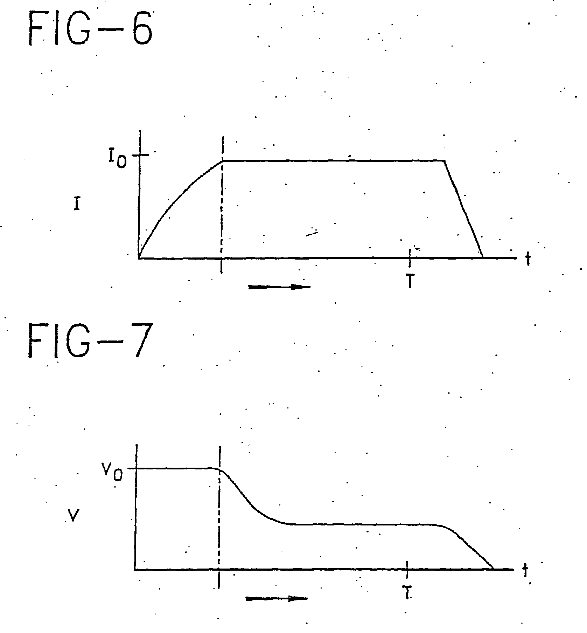

The device of the instant invention works as follows. An iontophoretic delivery device such as device 20 is attached to the skin of an animal. The attachment can be accomplished by removing the backing sheet and pressing the device against the skin.Next, electrical power is caused to flow between the electrodes at a constant voltage V₀ as illustrated in Figs. 3 and 7. V₀ is of a sufficiently low magnitude not to cause irritation to the skin. Because the initial high impedance of skin, the current is at a low value during this time as illustrated in Fig. 6. Because current is flowing, the skin impedance begins to fall allowing more current to flow. This in turn causes the skin impedance to decrease further, which permits more current to flow, and so on as illustrated in Fig. 6. At time T in Fig. 6 the current reaches the pre-selected value of I₀. When I₀ is reached, the electronic circuit, supplying a controlled voltage V₀ to the electrodes, as will be explained in more detail hereinafter, switches modes and becomes a constant current source (see Figs. 6 and 7). Fig. 6 illustrates the rise of current to I₀ at which time current becomes constant at I₀. When I₀ is reached, and the system delivers constant current I₀, the voltage begins falling. This falling off of voltage from the V₀ value is due to the decreasing resistance of the skin. The instant invention functions such that the voltage is prevented from rising to a level which can cause irreversible skin breakdown which leads to burns. At the same time, the desirable constant current capability is provided once the switchover to I₀ is achieved.

Accordingly, the instant invention may be used by attaching an iontophoretic drug delivery device to the skin, as described hereinabove, and to at least initially impress a constant voltage across the electrodes of the device. The current flowing through the electrodes is monitored while the constant voltage is applied. The electronic circuit senses when the monitored current reached a predetermined current, such as l₀, and switches from supplying a constant voltage to the electrodes to supplying a constant current, I₀. This current continues to flow between the electrodes, at a constant predetermined value, until the therapy regimen is completed.

It is known that there is a retationship between the current delivered to the electrodes and the quantity of ions transferred through the skin. Accordingly, the amount of current with respect to time can be measured to determine the amount of drug transferred through the skin. When using the method of the instant invention, the user can monitor the total current (over time) flowing between the electrodes and terminate power to the electrodes when the total amount of current, corresponding to the delivery of the desired amount of ionic compound into the skin, has been achieved.

It is known that high values of voltage and current will cause irritation and skin burning, As illustrated in Fig. 3, in some instances, the desirable power limit shown by the current designated P₀ is beyond the desirable values for V₀ and I₀. However, in other circumstances if the iontophoretic delivery was carried out at V₀ until the value of I₀ was reached, irritation may be caused to the skin. Accordingly, as best illustrated in Fig. 4, safeguards may be incorporated so that in carrying out the transition from constant voltage to constant current, a predetermined constant power P1 is not exceeded. The power supplied to the electrodes may be monitored throughout the delivery of current at constant voltage V₀. If the power reaches a predetermined value, the voltage may be lowered along the constant power curve P1 until the desired I₀ value is reached, as illustrated in Fig. 4. As will be explained in greater detail, circuitry may be included to implement the transition between constant voltage and constant current along a constant power curve.

One form of the electronic circuit for providing a controlled voltage, current or power to the electrodes of the drug delivery device 20 is shown in Fig. 5. The electronic circuit includes a set point selector circuit 50. Set point selector circuit 50 functions as an electronic, single pole, triple throw switch. The set point selector circuit includes: three inputs which are the poles of the switch; an output, which is effectively connected to the wiper of the switch; and a control input, which effectively controls the position of the wiper and its connection to one of the poles of the circuit.

Three predetermined voltage level signals are provided on the inputs of the set point selector circuit 50. A first voltage level is provided on a first input and represents a first power limit signal which is the maximum amount of power which is desired to be supplied to the electrodes of the drug delivery device 20. A second predetermined voltage level is provided to a second input of the set point selector circuit 50 and represents a first voltage limit signal, that is, the maximum voltage which is to be supplied to the electrodes of device 20. A third predetermined voltage level is provided to the third input of the set point selector circuit 50 and represents the desired delivery current, that is, the constant current which is desired to flow between the electrodes of device 20. One of these predetermined voltage level signals on the three inputs of the set point selector circuit 50 will be passed through the selector circuit to the output in response to a control signal provided to the control input of the set point selector circuit. Accordingly, the set point selector circuit 50 provides a set point signal on its output which is, effectively, a predetermined voltage level.

The electronic circuit further includes a feedback selector circuit 52. Feedback selector circuit 52 is similar in many respects to the set point selector circuit 50. It functions electronically as a single pole, triple throw switch, having three separate inputs which are the poles of the switch, a single output (connected effectively to the wiper of the switch), and a control input which effectively controls the position of the wiper and the connection between the output of the feedback selector circuit and one of its three inputs.

Three voltage levels are provided on the inputs of feedback selector circuit 52. The first voltage level is provided to the first input and is a sensed current feedback signal. The second voltage level is provided to the second input of the feedback selector circuit 52 and is a sensed voltage feedback signal. The third voltage level is provided to the third input and is a sensed power feedback signal. As will be explained in greater detail, each of these signals results from monitoring and measuring the current and voltage provided to the drug delivery device 20 by the electronic circuit. The output of the feedback selector circuit 52 provides a feedback signal which, as will be explained, is compared to the set point signal in a summer or subtractor circuit 53.

The voltage and current supplied to the drug delivery device 20 are monitored by differential amplifiers 54 and 56. The first differential amplifier 54 has each of its two inputs coupled to one of the electrodes of the transdermal drug delivery device 20. The output of the amplifier 54 provides a sensed voltage feedback signal, as described previously, which varies proportionally to the voltage supplied across the electrodes of device 20.

The second differential amplifier 56 has its two inputs connected across a current sense resistor 58 which is connected in series with one of the electrodes of device 20. The current flowing through the electrodes will also flow through current sense resistor 58, which is a fixed resistor of relatively low value, and will cause a voltage drop across the resistor, which is sensed by second differential amplifier 56. The output of the second differential amplifier 56 provides a sensed current feedback signal, as described previously. The sensed voltage and current feedback signals are provided to the respective inputs of feedback selector circuit 52.

The electronic circuit of the iontophoresis system further includes a multiplication circuit 60. Multiplication circuit 60 includes two inputs on which are provided the sensed current feedback signal and the sensed voltage feedback signal. The multiplication circuit 60 multiplies the two feedback signals and provides a sensed power feedback signal on its output The sensed power feedback signal is provided to one of the inputs of the feedback selector circuit 52, as described previously.

Set point selector circuit 50 and feedback selector circuit 52 are controlled by a set point and feedback control logic circuit 62. Set point and feedback control logic circuit 62 generates a control signal which is provided to the control inputs of each of the set point selector circuit 50 and feedback selector circuit 52. The set point and feedback control logic circuit 62 may, in one form, be a read only memory (ROM), having preferably three inputs. On one input is provided a current state signal, and on another input is provided a power state signal, and on a third input is provided a voltage state signal. Each of the current state signal, and voltage state signal and power state signal is preferably a logic signal, and the control signal generated by the set point and feedback control logic circuit 62 is also preferably a logic signal.

The electronic circuit of the iontophoresis system further includes a first and second comparator 64 and 66, and in a preferred form of the invention, a third comparator 67. First comparator 64 generates the current state signal on its output, and second comparator 66 generates the power state signal on its output. Third comparator 67 generates the voltage state signal on its output. First comparator 64 is provided with a current limit signal, which may be in the form of a selectable DC voltage, on one of its inputs. The current limit signal may be equal to the desired delivery current signal provided to the set point selector circuit 50. The other input of the first comparator 64 is provided with the sensed current feedback signal.

Similarly, second comparator 66 is provided with a second power limit signal, which may be in the form of a preselected voltage level, on one of its inputs. The second power limit signal may be equal to the first power limit signal provided to the set point selector circuit 50. The other input of the second comparator 66 is provided with the sensed power feedback signal generated by multiplier circuit 60.

The third comparator 67 is provided with a second voltage limit signal, which may be in the form of a preselected voltage level, on one of its outputs. The second voltage limit signal may be equal to the first voltage limit signal provided to the set point selector circuit 50. The other input of the third comparator 67 is provided with the sensed voltage feedback signal.

The current state signal will be at one logic level when the current limit signal is greater than the sensed current feedback signal provided to the first comparator 64, and will be in a second logic state when the current limit signal is less than or equal to the sensed current feedback signal. Similarly, the power state signal will be in one logic state when the second power limit signal is greater than the sensed power feedback signal provided to the second comparator 66, and will be in a different state when the power limit signal is less than or equal to the sensed power feedback signal. The voltage state signal will be in one logic state when the second voltage limit signal is greater than the sensed voltage feedback signal provided to the third comparator 67, and will be in a different state when the voltage limit signal is less than or equal to the sensed voltage feedback signal.

Set point and feedback control logic circuit 62 is preprogrammed to provide different control signals in response to the different conditions of the current state signal and the second power state signal and, if the third comparator 67 is included in the second voltage limit signal. If the sensed current feedback signal and the sensed power feedback signal are respectively below the current limit signal and the second power limit signal provided to the first and second comparators 64 and 66, set point and feedback control logic circuit 62 will generate a control signal which will cause set point selector circuit 50 and feedback selector circuit 52 to provide respectively a set point signal and feedback signal which are equal or correspond to the first voltage limit signal (provided to the set point selector circuit 50) and the sensed voltage feedback signal (provided to the feedback selector circuit 52). If the sensed current feedback signal is below the current limit signal provided to first comparator 64, and the sensed power feedback signal is greater than or equal to the second power limit signal provided to second comparator 66, set point and feedback control logic circuit 62 will provide a control signal which will cause set point selector circuit 50 and feedback selector circuit 52 to provide respectively a set point signal and a feedback signal which are equal or correspond to the first power limit signal provided to the set point selector circuit 50 and the sensed power feedback signal provided to feedback selector circuit 52.

If the sensed current feedback signal is greater than or equal to the current limit signal provided to first comparator 64, and the sensed power feedback signal is less than the second power limit signal provided to the second comparator 66, the set point and feedback control logic circuit 62 will provide a control signal to the set point selector circuit 50 and the feedback selector circuit 52 such that the set point selector circuit and the feedback selector circuit will respectively generate a set point signal and feedback signal which are equal or correspond to the desired delivery current signal provided to the set point selector circuit 50 and the sensed current feedback signal provided to feedback selector circuit 52. If the sensed current feedback signal is greater than or equal to the current limit signal provided to the first comparator 64, and the sensed power feedback signal is greater than or equal to the power limit signal provided to second comparator 66, set point and feedback control logic circuit 62 will generate a control signal which will cause the set point selector circuit and the feedback selector circuit to generate respectively a set point signal and a feedback signal which are equal or correspond to the first power limit signal provided to set point selector circuit 50 and the sensed power feedback signal provided to feedback selector, circuit 52.

It is possible that the impedance of the patient's skin may increase while the transdermal delivery system is operating in the constant current mode. This will cause the voltage delivered to the patient to increase, possibly causing discomfort, or even injury if a power limit is not used. Under such circumstances, it may be desirable for the circuit to switch back into a controlled, constant voltage mode, The third comparator 67 may be included for such a purpose. Thus, if the sensed voltage feedback signal equals or exceeds the second voltage limit provided to the third comparator 67, the set point and feedback control logic circuit 62 will provide a control signal to the set point selector circuit 50 and the feedback selector circuit 52 such that the set point selector circuit and the feedback selector circuit will respectively generate a set point signal and feedback signal which are equal or correspond to the first voltage limit signal (provided to the set point selector circuit 50) and the sensed voltage feedback signal (provided to the feedback selector circuit 52).

Of course, it should be realized that the set point and feedback control circuit 62 will cause the delivery system to go into a cortrolled constant power mode if the sensed power feedback signal equals or exceeds the second power limit signal provided to the second comparator 66. This will occur whether or not the current limit signal or the second voltage limit signal is exceeded.

The following table illustrates the mode which the circuit will be in response to whether the sensed current, voltage and power feedback signals are above or below their respective threshold limits provided to comparators 64, 66 and 67.

**TABLE 1**

| C | V | P | MODE |
|---|---|---|---|
| Below Limit | Below Limit | Below Limit | Voltage |
| Above or Equal to Limit | Below Limit | Below Limit | Current |
| Below Limit | Above or Equal to Limit | Below Limit | Voltage |
| Above or Equal to Limit | Above or Equal to Limit | Below Limit | Voltage |
| X | X | Above or Equal to Limit | Power |
| C = sensed current feedback signal | | | |
| V = sensed voltage feedback signal | | | |
| P = sensed power feedback signal | | | |
| X = "don't care" condition | | | |

It should be noted that the power mode may be omitted and its related structure such as comparator 66, multiplication circuit 60 and the first and second power limit signals. The circuit would include the structure described previously, including comparators 64 and 67, which would allow the circuit to switch between a controlled, constant current mode and a controlled constant voltage mode.

As mentioned previously, the set point signal and feedback signal are provided to a summer or subtractor circuit 53. Summer or subtractor circuit 53 will subtract the feedback signal from the set point signal and provide an error signal on its output, which error signal corresponds to the difference in voltage levels between the set point signal and the feedback signal.

The error signal from the summer circuit 53 is, in one form of the invention, provided to the input of a variable pulse generator 68, such as a voltage controlled osdllator (VCO). Variable pulse generator 68 provides a pulsed output signal which varies in either frequency or duty cycle proportionally to the magnitude of the error signal generated by summer circuit 53.

The electronic circuit of the iontophoresis system further includes a pulse to power converter circuit 70. Pulse to power converter circuit 70 includes an input to which is provided the output signal from variable pulse generator 68, and a pair of outputs across which is a controlled voltage which is generated by the pulse to power converter circuit from a power source 72 connected to another input of the converter circuit 70. The output voltage generated by pulse to power converter circuit 70 across its outputs will vary in accordance with the frequency or duty cycle of the pulsed output signal from the pulse generator 68 and, in turn, the voltage level of the error signal generated by summer circuit 53.

An alternative embodiment of the system circuit is shown in Fig. 5A. The pulse generator 68 and pulse to power converter circuit 70 may be replaced with a power amplifier circuit 73. The power amplifier circuit 73 includes an input which receives the error signal from summer circuit 53, and generates an output voltage which varies in response to the error signal. The output voltage is provided to the drug delivery device 20 in a manner similar to that which is described in relation to the embodiment of Fig. 5.

The portion of the electronic drcuit of the iontophoresis system thus described provides a selectable, controlled constant voltage, constant current or constant power to the electrodes of the transdermal drug delivery device 20, as described previously, through feedback When the iontophoresis system is initially applied to the patient, the current sensed by the circuit flowing through the electrodes will be at a relatively low level, that is, less than the l₀ threshold, because of the relatively high resistance of the patient's tissue. Under such conditions, the electronic circuit will be in a constant voltage mode so as to provide a constant voltage to transdermal delivery device 20. The sensed voltage feedback and the voltage limit signal will, effectively, be compared in summer circuit 53, and any differences between the two will be adjusted for by pulse to power converter drcuit 70 (or power amplifier circuit 73, as shown in Fig. 5A).

When the sensed current feedback signal rises to the level of the current limit signal, because of the decrease in the resistance to the patient's tissue, the electronic circuit will switch into a constant current mode. The control signal generated by set point and feedback control logic circuit 62 will cause the delivery current signal and the sensed current feedback signal to be provided to summer or subtractor circuit 53 in order to cortrol variable pulse generator 68 driving pulse to power converter circuit 70. Any differences between the desired delivery current signal and the sensed current feedback signal will be adjusted for by pulse to power converter circuit 70 and the feedback loop of the electronic circuit.

If, either in the constant voltage mode or the constant current made, the sensed power feedback signal equals or exceeds the second power limit signal, set point and feedback control logic circuit 62 will generate a control signal such that the electronic circuit will go into a constant power mode. The control signal will cause the first power limit signal and the sensed power feedback signal to be provided to the inputs of summer or subtractor circuit 53, and any differences between the two will be adjusted for by pulse to power converter circuit 70 (or power amplifier circuit 73) and the feedback loop of the electronic circuit.

The iontophoresis system according to an advantageous embodiment of the present invention indirectly monitors and measures the total quantity of the drug provided transdermally to the patent, and will stop delivery when a selectable, predetermined total dosage level is reached. More specifically, the electronic circuit includes an output enable switch circuit 74 in the form of an electronic, single pole, single throw switch, which is connected between one output of the pulse to power converter circuit 70 (or amplifier 73 in the embodiment of Fig. 5A) and one of the electrodes of the drug delivery device 20. The position of the "wiper* of output enable switch circuit 74 is controlled by a dose state signal provided to the control input of the switch. Preferably, the dose state signal is a logic signal which is generated by a fourth comparator 76 on its output, and is provided to switch circuit 74 partially along dashed line 78, which bypasses an output enable logic circuit 80 of an alternative form of the present invention, as will be described in greater detail.

Fourth comparator 76 includes at least two inputs. A total dosage signal, which may be in the form of a preselected voltage level, is provided on one input, and an accumulated current signal, which may also be in the form of a variable voltage level, is provided to the other input of the fourth comparator 76.

The electronic circuit further indudes an accumulator circuit 82. The accumulator circuit 82 has an input on which is provided the sensed current feedback signal, an output on which is provided the accumulated current signal. Accumulator circuit 82 effectively Integrates and accumulates the sensed current feedback signal over time. It may be in the form of a circuit in which the sensed current feedback signal is converted from a voltage to a current which accumulates charge on a capacitor, the resulting voltage across the capacitor increasing in proportion to the current flowing into it. The voltage across the capacitor may be used in generating the accumulated current signal.

The accumulated current signal, which is in the form of a variable voltage level, is proportional to the current flowing through the electrodes of the transdermal drug delivery device 20, which current, in tum, is representative of the total drug dose administered to the patient If the accumulated current signal is below the total dose signal, the dose state signal generated by fourth comparator 76 will be in one state and will cause output enable switch circuit 74 to maintain the connection between pulse to power converter circuit 70 (or amplifier 73) and one of the electrodes of delivery device 20. If the accumulated current signal equals or exceeds the total dose signal, the dose state signal will be in another state which will cause the output enable switch circuit 74 to open, disconnecting the pulse to power converter circuit 70 (or amplifier 73) from drug delivery device 20. Accordingly, the electronic circuit of the iontophoresis system will automatically disconnect power to drug delivery device 20 when the proper drug dosage has been administered to the patient.

In a preferred form of the present invention, the electronic circuit may further include an over-current shut down comparator circuit This circuit disables the output of the pulse to power converter circuit 70 (or amplifier 73) in the event that the sensed current feedback signal exceeds a predetermined level, which preferably corresponds to 1.5 times the current delivered to the electrodes of drug delivery device 20.

More specifically, the over-current shut down comparator circuit indudes an output enable logic circuit 80, which may be in the form of a ROM, which is interposed between the output of the fourth comparator 76 and the control input of output enable switch circuit 74. The output enable logic circuit 80 provides an output enable signal to output enable switch circuit 74 to control the position of the switch circuit in much the same way as the dose state signal from fourth comparator 76 did in the previous embodiment

The dose state signal from fourth comparator 76, In this embodiment, is provided to one input of output enable logic circuit 80. An excess current limit signal, which may be in the form of a logic signal, is provided to another output of output enable logic circuit 80. The excess current limit signal is generated by a fifth comparator 82 on its output.

Fifth comparator 82 includes two inputs. One input is provided with an excess current threshold signal, which may be in the form of a pre-solected voltage level which may be equal to 1.5 times the voltage level of the desired delivery current signal. The second input is provided with the sensed current feedback signal.

The output enable logic circuit 80 will control the state of output enable switch circuit 74 In response to whether a total dose has been administered to the patient or whether the instantaneous, sensed current feedback signal exceeds the excess current threshold signal. More specifically, if the accumulated current signal is less than the total dose signal, the output enable signal generated by output enable logic circuit 80 will be such as to cause output enable switch circuit 74 to maintain a connection between the pulse to power converter circuit 70 (or amplifier circuit 73) and drug delivery device 20.

Irrespective of whether the total dose has been administered to the patient, if the sensed current feedback signal equals or exceeds the excess current threshold signal, fifth comparator 82 will provide an output signal to output enable logic circuit 80 which, in tum, will cause the output enable signal to open the output enable switch circuit 74, thereby disconnecting the pulse to power converter circuit 70 (or amplifier 73) from the drug delivery device 20. If, of course, the excess current threshold signal has not been exceeded or reached try the sensed current feedback signal and the total dose signal is greater than the accumulated current signal, the output enable logic circuit 80 will cause the output enable switch circuit 74 to maintain a connection between the pulse to power converter circuit 70 (or amplifier 73) and the drug delivery device 20. Thus, the electronic circuit of the lontophoresis system will automatically disconnect power from drug delivery device 20 if either the total dose has been administered to the patient or an excess current condition has been detected.

The circuits shown in Fig. 5 and 5A and described previously provide a controlled, constant direct current or voltage to the transdermal drug delivery device 20.

The skin impedance at which one desires to switch modes is dependent upon a number of factors, including the effective area of the drug reservoir 21 in contact with the patient's skin, the magnitude of the constant current delivered to the drug reservoir over the effective skin contact area, and the voltage across the electrodes 22,25 of the drug delivery device at the time of switching from the constant voltage mode to the constant current mode.

For example, if the drug reservoir skin contact area is 5 square centimeters and the current provided to the drug reservoir is 200 µa/square cm (for a total current of 1 ma), and the voltage across the electrodes at the time of switching modes is 6 volts, then the predetermined skin impedance is 6,000 ohms.

If, on the other hand, a 1 square centimeter (contact area) "patch" is employed which is driven by a current of 100 µa/square cm (for a total current .1 ma), and the voltage across the electrodes at the time of switching modes is 20 volts, then the predetermined skin impedance is 200K ohms for switching.

It has been found that the voltage during transdermal drug delivery treatment should be between about 3 and about. 30 volts and, more preferably, between about 6 and about 20 volts to achieve effective drug delivery rates while avoiding discomfort to the patient.

## Claims

1. An operable iontophoretic drug delivery system comprising a drug reservoir (21) adapted to be attached to the skin of an animal wherein said drug reservoir (21) contains an ionic compound selected from the group consisting of therapeutic compounds, diagnostic compounds and drugs, a first electrode (22) in said drug reservoir (21), a second electrode (25) adapted to be brought into electrical connection with said animal, an electrolyte reservoir (23) adapted to be placed in communication with the skin of the animal, said second electrode (25) being positioned within the electrolyte reservoir, circuit means for providing electrical communication between said first and second electrodes (22, 2,5), said circuit means including means for connecting to a source of electrical power, said circuit means comprising a first supply means for supplying controlled voltage between said electrodes (22, 25) when the system is activated, said circuit means further comprising a second Supply means for providing controlled current to one of said electrodes (22), said circuit means further including means for monitoring the current through said one of said electrodes (22), and switching means in said circuit means,
**characterised by**
said switching means being operable for switching from a first mode wherein said first supply means supplies said controlled voltage as a controlled constant direct voltage ranging between 3 and 30 Volts to a second mode wherein said second supply means provides said controlled current as a controlled constant direct current, when said current monitored by said monitoring means reaches a predetermined value,

2. The operable iomopheresis drug delivery system of claim 1,
wherein said first electrode (22) includes a conductive element selected from the group consisting of silver, silver/chloride, gold, copper, molybdenum and lead.

3. The operable iontophoresis drug delivery system of one of claims 1 or 2 further including means for measuring the current passing through said first electrode (22) and for accumulating the total current passing through said first electrode.

4. The operable iontophoresis drug delivery system of claim 3 further including means for terminating power to said first electrode (22) when accumulated total current reaches a predetermined value.

5. The operable iontophoretic drug delivery system according to one of claims 1-4 wherein the circuit means comprises an electronic circuit, said electronic circuit comprising:
adjustable voltage and current generating means (68,70) coupled to the electrodes (22,25) of the drug delivery system (20) and providing one of a selectively adjustable current and voltage to the electrodes (22,25)
means for sensing the current (56,58) provided to the electrodes (22,25) of the drug delivery system (20), the signal which is proportional to the sensed electrode current,
means (54) for sensing voltage supplied the eledrodes of the drug delivery system (20), the voltage sensing means (54) generating a sensed voltage feedback signal which is proportional to the sensed electrode voltage,
means (64) coupled to the current sensing means (56, 58) for comparing the sensed current feedback signal with a predetermined current threshold signal and generating a current state signal in response to the comparison thereof,
feedback signal selector means (52), the feedback signal selector means (52) being responsive to at least the current state signal and receiving at least the sensed current feedback signal and sensed voltage feedback signal and generating a feedback signal corresponding to at least one of the sensed current feedback signal and the sensed voltage feedback signal in response to at least the current state signal,
setpoint selector means (50), the setpoint selector means (50) receiving a preselected voltage limit signal and preselected desired drug delivery current signal and being responsive to at least the current state signal, the setpoint selector means (50) generating a setpoint signal corresponding to at least one of the voltage limit signal and the desired delivery current signal in response to at least the current state signal,
means (53) for subtracting the feedback signal from the setpoint signal, the subtracting means generating an error signal corresponding to the difference between the feedback signal and the setpoint signal, and
the voltage and current generating means (68,70) being responsive to the error signal and adjusting at least one of the current and voltage provided to the electrodes (22;25) in response thereto.

6. The operable iorrtophoretic drug delivery system of claim 5 wherein the adjustable voltage and current generating means (68,70) includes a variable pulse generator (68) and a pulse to power converter (70) coupled to the variable pulse generator (68), the variable pulse generator (68) being responsive to the error signal and generating a pulsed output signal which varies in correspondence to the error signal, the pulse to power converter (70) being responsive to the output signal of the variable pulse generator (68) and generating an output voltage which varies in correspondence to the pulse generator (68) output signal.

7. The operable iontophoretic drug delivery system of one of claims 5 or 6 wherein the electronic circuit further comprises:
output enable switch means (74), the output enable switch means (74) being coupled to the circuit in series between one of the electrodes (22,25) of the drug delivery system (20) and the voltage and current generating means (68,70), the output enable switch means (74) being responsive to a dose state signal and selectively interrupting current flow provided to said one of the electrodes (22.25) in response thereto,
total drug dose sensing means (76), the total drug dose sensing means (76) receiving a predetermined total dose signal and comparing the total dose signal with a signal substantially representative of the quantity of drug administered to the patient by the drug delivery system (20) and generating the dose state signal in response to the comparison thereof, and
means (84) for determining the quantity of current over time provided to the electrodes (22,25) of the drug delivery system (20), the current quantity de-termining means (84) providing an accumulated current signal in response thereto, the accumulated current signal being the signal substantially representative of the quantity of drug administered to the patient by the drug delivery system (20).

8. The operable iontophoretic drug delivery system of one of claims 5-7 wherein the efectronic circuit further comprises:
output enable switch means (74), the output enable switch means (74) being coupled to the circuit in series between one of the electrodes (22,25) of the drug delivery system (20) and the voltage and current generating means (68,70), the output enable switch means (74) being responsive to an excess current limit signal and selectively interrupting current flow provided to said one at the electrodes (22,25) in response thereto, and
means (82) for comparing the sensed current feedback signal with a predetermined excess current threshold signal and generating the excess current limit signal in response to the comparison thereof.

## Patentansprüche

1. Betreibbares iontophoretisches Medikamentenzufuhrsystem umfassend ein Medikamentenreservoir (21), das an der Haut eines Tieres angebracht werden kann, wobei das Medikamentenreservoir (21) eine ionische Verbindung ausgewählt aus der Gruppe bestehend aus therapeutischen Verbindungen, diagnostischen Verbindungen und Medikamenten enthält, eine erste Elektrode (22) in dem Medikamentenreservoir (21), eine zweite Elektrode (25), die in elektrischer Verbindung mit dem Tier gebracht werden kann, ein Elektrolytreservoir (23), das angepasst ist, um in Verbindung mit der Haut des Tiers gebracht zu werden, wobei die zweite Elektrode (25) in dem Elektrolytreservoir angeordnet ist, eine Schaltungseinrichtung zur Bereitstellung einer elektrischen Verbindung zwischen der ersten und zweiten Elektrode (22, 25), wobei die Schaltungseinrichtung eine Einrichtung zum Verbinden mit einer elektrischen Stromquelle beinhaltet, die Schaltungseinrichtung eine erste Versorgungseinrichtung zur Lieferung einer gesteuerten Spannung zwischen den Elektroden (22, 25), wenn das System in Betrieb gesetzt ist, umfasst, die Schaltungseinrichtung ferner eine zweite Versorgungseinrichtung zur Bereitstellung von gesteuertem Strom an einer der Elektroden (22) umfasst, die Schaltungseinrichtung ferner eine Einrichtung zur Überwachung des Stroms durch die eine der Elektroden (22) beinhaltet, und eine Umschalteinrichtung in der Schaltungseinrichtung,
**dadurch gekennzeichnet, dass**
die Umschalteinrichtung betreibbar ist, um von einem ersten Modus, bei dem die erste Versorgungseinrichtung die gesteuerte Spannung als gesteuerte konstante direkte Spannung im Bereich zwischen 3 und 30 Volt liefert, zu einem zweiten Modus, bei dem die zweite Versorgungseinrichtung den gesteuerten Strom als gesteuerten konstanten direkten Strom bereitstellt, zu schalten, wenn der durch die Überwachungseinrichtung überwachte Strom einen vorbestimmten Wert erreicht.

2. Betreibbares iontophoretisches Medikamentenzufuhrsystem nach Anspruch 1, bei dem die erste Elektrode (22) ein leitfähiges Element ausgewählt aus der Gruppe bestehend aus Silber, Silber/Chlorid, Gold, Kupfer, Molybdän und Blei beinhaltet.

3. Betreibbares iontophoretisches Medikamentenzufuhrsystem nach einem der Ansprüche 1 oder 2, das ferner eine Einrichtung zum Messen des durch die erste Elektrode (22) fließenden Stroms und zum Akkumulieren des durch die erste Elektrode fließenden Gesamtstroms beinhaltet.

4. Betreibbares iontophoretisches Medikamentenzufuhrsystem nach Anspruch 3, das ferner eine Einrichtung zum Unterbrechen des Stroms zur ersten Elektrode (22), wenn der akkumulierte Gesamtstrom einen vorbestimmten Wert erreicht, beinhaltet.

5. Betreibbares iontophoretisches Medikamentenzufuhrsystem nach einem der Ansprüche 1 bis 4, bei dem die Schaltungseinrichung einen elektronischen Schaltkreis umfasst, wobei der elektronische Schaltkreis umfasst:
eine einstellbare Spannungs- und Stromerzeugungseinrichtung (68, 70), die mit den Elektroden (22, 25) des Medikamentenzufuhrsystems (20) verbunden ist und einen selektiv einstellbaren Strom oder eine selektiv einstellbare Spannung an die Elektroden (22, 25) liefert,
eine Einrichtung (56, 58) zum Detektieren des an die Elektroden (22, 25) des Medikamentenzufuhrsystems (20) gelieferten Stroms, wobei die Stromdetektionseinrichtung (56, 58) ein Rückkopplungssignal des detektierten Stroms erzeugt, das proportional zu dem detektierten Elektrodenstrom ist, eine Einrichtung (54) zum Detektieren der an die Elektroden des Medikamentenzufuhrsystems (20) gelieferten Spannung, wobei die Spannungsdetektionseinrichtung (54) ein Rückkopplungssignal der detektierten Spannung erzeugt, das proportional zu der detektierten Elektrodenspannung ist, eine Einrichtung (64), die mit der Stromdetektionseinrichtung (56, 58) verbunden ist, zum Vergleichen des Stromrückkopplungssignals des detektierten Stroms mit einem vorbestimmten Sromschwellenwertsignal und zum Erzeugen eines Stromzustandsignals als Antwort auf den Vergleich, eine Rückkopplungssignal-Auswahleinrichtung (52), wobei die Rückkopplungssignal-Auswahleinrichtung (52) zumindest auf das Stromzustandssignal reagiert und zumindest das Rückkopplungssignal des detektierten Stroms und das Rückkopplungssignal der detektierten Spannung empfängt und als Antwort auf zumindest das Stromzustandsignal ein Rückkopplungssignal erzeugt, das zumindest einem von dem Rückkopplungssignal des detektierten Stroms und dem Rückkopplungssignal der detektierten Spannung entspricht,
eine Sollwert-Auswahleinrichtung (50), wobei die Sollwert-Auswahleinrichtung (50) zumindest ein vorgewähltes Spannungsgrenzsignal und ein vorgewähltes gewünschtes Medikamentenzufuhr-Stromsignal empfängt und zumindest auf das Stromzustandssignal reagiert, wobei die Sollwert-Auswahleinrichtung (50) als Antwort auf zumindest das Stromzustandsignal ein Sollwertsignal erzeugt, das zumindest einem von dem Spannungsgrenzsignal und dem gewünschten Zufuhrstromsignal entspricht,
eine Einrichtung (53) zur Subtraktion des Rückkopplungssignals von dem Sollwertsignal, wobei die Subtraktionseinrichtung ein Fehlersignal erzeugt, das der Differenz zwischen dem Rückkopplungssignal und dem Sollwertsignal entspricht, und
die Spannungs- und Stromerzeugungseinrichtung (68, 70) auf das Fehlersignal reagiert und als Antwort darauf mindestens einen von dem an die Elektroden (22, 25) gelieferten Strom und der an die Elektroden (22, 25) gelieferten Spannung einstellt.

6. Betreibbares iontophoretisches Medikamentenzufuhrsystem nach Anspruch 5, wobei die einstellbare Spannungs- und Stromerzeugungseinrichtung (68, 70) einen variablen Impulsgenerator (68) und einen mit dem variablen Impulsgenerator (68) verbundenen Impuls-Leistungs-Wandler (70) beinhaltet, wobei der variable Impulsgenerator (68) auf das Fehlersignal reagiert und ein Impuls-Ausgangssignal erzeugt, das dem Fehlersignal entsprechend variiert, wobei der Impuls-Leistungs-Wandler (70) auf das Ausgangssignal des variablen Impulsgenerators (68) reagiert und eine Ausgangsspannung erzeugt, die entsprechend dem Ausgangssignal des Impulsgenerators (68) variiert.

7. Betreibbares iontophoretisches Medikamentenzufuhrsystem nach einem der Ansprüche 5 oder 6, bei dem der elektronische Schaltkreis ferner umfasst:
eine Ausgangsfreigabe-Schalteinrichtung (74), wobei die Ausgangsfreigabe-Schalteinrichtung (74) mit dem Schaltkreis zwischen einer der Elektroden (22, 25) des Medikamentenzufuhrsystems (20) und der Spannungs-und Stromerzeugungseinrichtung (68, 70) in Reihe geschaltet ist, wobei die Ausgangsfreigabe-Schalteinrichtung (74) auf ein Dosiszustandssignal reagiert und als Antwort darauf selektiv den an die eine der Elektroden (22, 25) gelieferten Stromfluss unterbricht,
eine Medikamentengesamtdosis-Detektionseinrichtung (76), wobei die Medikamentengesamtdosis-Detektionseinrichtung (76) ein vorbestimmtes Gesamtdosissignal empfängt und das Gesamtdosissignal mit dem Signal vergleicht, das im wesentlichen die dem Patienten durch das Medikamentenzufuhrsystem (20) verabreichte Medikamentenmenge darstellt und als Antwort auf den Vergleich ein Dosiszustandssignal erzeugt, und
eine Einrichtung (84) zum Bestimmen des über einen Zeitraum an die Elektroden (22, 25) des Medikamentenzufuhrsystems (20) gelieferten Stroms, wobei die Strommengenbestimmungseinrichtung (84) als Antwort ein akkumuliertes Stromsignal liefert, wobei das akkumulierte Stromsignal das Signal ist, das im wesentlichen die dem Patienten durch das Medikamentenzufuhrsystem (20) verabreichte Medikamentenmenge darstellt.

8. Betreibbares iontophoretisches Medikamentenzufuhrsystem nach einem der Ansprüche 5 bis 7, bei dem der elektronische Schaltkreis ferner umfasst:
eine Ausgangsfreigabe-Schalteinrichtung (74), wobei die Ausgangsfreigabe-Schalteinrichtung (74) mit dem Schaltkreis zwischen einer der Elektroden (22, 25) des Medikamentenzufuhrsystems (20) und der Spannungs-und Stromerzeugungseinrichtung (68, 70) in Reihe geschaltet ist, wobei die Ausgangsfreigabe-Schalteinrichtung (74) auf ein Überschussstromgrenzsignal reagiert und als Antwort darauf selektiv den an die eine der Elektroden (22, 25) gelieferten Stromfluss unterbricht, und
eine Einrichtung (84) zum Vergleichen des Rückkopplungssignals des detektierten Stroms mit einem vorbestimmten Überschussstromschwellenwertsignal und zum Erzeugen des Überschussstromgrenzsignals als Antwort auf den Vergleich.

## Revendications

1. Dispositif d'administration de médicament par ionophorèse réglable, comprenant un réservoir de médicament (21) conçu pour être fixé sur la peau d'un animal, dans lequel ledit réservoir de médicament (21) contient un composé ionique sélectionné à partir du groupe consistant en composés thérapeutiques, composés de diagnostics et médicaments, une première électrode (22) dans ledit réservoir de médicament (21), une seconde électrode (22) conçue pour être portée en contact électrique avec ledit animal, un réservoir d'électrolyte (23) conçu pour être placé en communication avec la peau de l'animal, ladite seconde électrode (25) étant disposée à l'intérieur du réservoir d'électrolyte, un moyen formant circuit destiné à assurer une communication électrique entre lesdites première et seconde électrodes (22, 25), ledit moyen formant circuit comprenant un moyen de connexion à une source de puissance électrique, ledit moyen formant circuit comprenant un premier moyen d'alimentation destiné à délivrer une tension commandée entre lesdites électrodes (22, 25) lorsque le dispositif est activé, ledit moyen formant circuit comprenant en outre une second moyen d'alimentation destiné à délivrer un courant commandé à l'une (22) desdites électrodes, ledit moyen formant circuit comprenant en outre un moyen destiné à surveiller le courant à travers ladite électrode (22), et un moyen de commutation dans ledit moyen formant circuit,
**caractérisé en ce que**
ledit moyen de commutation est actionnable pour commuter d'un premier mode dans lequel ledit premier moyen d'alimentation délivre ladite tension commandée sous la forme d'une tension continue, constante, commandée, dans l'intervalle de 3 à 30 Volts, à un second mode dans lequel ledit second moyen d'alimentation délivre ledit courant commandé sous la forme d'un courant continu, constant, commandé, lorsque ledit courant surveillé par ledit moyen de surveillance atteint une valeur prédéterminée.

2. Dispositif d'administration de médicament par ionophorèse réglable selon la revendication 1, dans lequel ladite première électrode (22) comprend un organe conducteur sélectionné parmi le groupe formé de l'argent, de l'argent/chlorure, de l'or, du cuivre, du molybdène et du plomb.

3. Dispositif d'administration de médicament par ionophorèse réglable selon l'une des revendications 1 et 2, comprenant en outre un moyen destiné à mesurer le courant passant à travers ladite première électrode (22) et à cumuler le courant total passant à travers ladite première électrode.

4. Dispositif d'administration de médicament par ionophorèse réglable selon la revendication 3, comprenant en outre un moyen destiné à couper la puissance sur ladite première électrode (22) lorsque le courant total cumulé atteint une valeur prédéterminée.

5. Dispositif d'administration de médicament par ionophorèse réglable selon l'une des revendication 1 à 4, dans lequel le moyen formant circuit est constitué par un circuit électronique, ledit circuit électronique comprenant:
un moyen (68, 70) de production de tension et de courant réglables couplé aux électrodes (22, 25) du dispositif d'administration de médicament (20) et délivrant l'un parmi un courant et une tension réglables de manière sélective aux électrodes (22, 25),
un moyen (56, 58) destiné à détecter le courant délivré aux électrodes (22, 25) du dispositif d'administration de médicament (20), le moyen (56, 58) de détection de courant produisant un signal d'asservissement de courant détecté qui est proportionnel au courant d'électrode détecté,
un moyen (54) destiné à détecter la tension délivrée aux électrodes du dispositif d'administration de médicament (20), le moyen de détection de tension (54) produisant un signal d'asservissement de tension détectée qui est proportionnel à la tension d'électrode détectée,
un moyen (54) couplé au moyen (56, 58) de détection de courant pour comparer le signal d'asservissement de courant détecté avec un signal de seuil de courant prédéterminé et produire un signal d'état de courant en fonction de leur comparaison,
un moyen (52) de sélection de signal d'asservissement, le moyen (52) de sélection de signal d'asservissement étant sensible au moins au signal d'état de courant et recevant au moins le signal d'asservissement de courant détecté et le signal d'asservissement de tension détectée et produisant un signal d'asservissement correspondant à au moins l'un parmi le signal d'asservissement de courant détecté et le signal d'asservissement de tension détectée au moins en fonction du signal d'état de courant,
un moyen (50) de sélection de point de consigne, le moyen (50) de sélection de point de consigne recevant un signal de limite de tension présélectionné et un signal de courant d'administration de médicament désiré et étant sensible au moins au signal d'état de courant, le moyen (50) de sélection de point de consigne produisant un signal de point de consigne correspondant à l'un au moins parmi le signal de limite de tension et le signal de courant d'administration désiré, au moins en fonction du signal d'état de courant
un moyen (53) destiné à soustraire le signal d'asservissement du signal de consigne, le moyen de soustraction produisant un signal d'erreur correspondant à la différence entre le signal d'asservissement et le signal de consigne, et
le moyen (68, 70) de production de tension et de courant étant sensible au signal d'erreur et réalisant le réglage d'au moins l'un parmi le courant et la tension délivrés aux électrodes (22, 25) en fonction celui-ci.

6. Dispositif d'administration de médicament par ionophorèse réglable selon la revendication 5, dans lequel le moyen (68, 70) de production de tension et de courant réglables comprend un générateur d'impulsion variable (68) et un convertisseur d'impulsion en puissance (70) couplé au générateur d'impulsion variable (68), le générateur d'impulsion variable (68) étant sensible au signal d'erreur et produisant un signal de sortie impulsionnel qui varie en fonction du signal d'erreur, le convertisseur d'impulsion en puissance (70) étant sensible au signal de sortie du générateur d'impulsion variable (68) et produisant une tension de sortie qui varie en fonction du signal de sortie du générateur d'impulsion (68).

7. Dispositif d'administration de médicament par ionophorèse réglable selon l'une des revendications 5 et 6, dans lequel le circuit électronique comprend en outre un moyen (74) de commutation de validation de sortie, le moyen (74) de commutation de validation de sortie étant couplé au circuit, en série entre l'une des électrodes (22, 25) du dispositif d'administration de médicament (20) et le moyen (68, 70) de production de tension et de courant, le moyen (74) de commutation de validation de sortie étant sensible à un signal d'état de dose et interrompant de manière sélective le passage du courant délivré à ladite électrode (22, 25) en fonction de celui-ci,
un moyen (76) de détection de dose totale de médicament, le moyen (76) de détection de dose totale de médicament recevant un signal de dose totale prédéterminé et comparant le signal de dose totale avec un signal sensiblement représentatif de la quantité de médicament administrée au patient par le dispositif d'administration de médicament (20) et produisant le signal d'état de dose en fonction de leur comparaison, et
un moyen (84) destiné à déterminer la quantité de courant en fonction du temps délivré aux électrodes (22, 25) du dispositif d'administration de médicament (20), le moyen (84) de détermination de la quantité de courant délivrant un signal de courant cumulé en fonction de celle-ci, le signal de courant cumulé étant le signal sensiblement représentatif de la quantité de médicament administrée au patient par le dispositif d'administration de médicament (20).

8. Dispositif d'administration de médicament par ionophorèse réglable selon rune des revendications 5 à 7, dans lequel le circuit électronique comprend en outre:
un moyen (74) de commutation de validation de sortie, le moyen (74) de commutation de validation de sortie étant couplé au circuit, en série entre l'une des électrodes (22, 25) du dispositif d'administration de médicament (20) et le moyen (68, 70) de production de tension et de courant, le moyen (74) de commutation de validation de sortie étant sensible à un signal de limite d'excès de courant et interrompant de manière sélective le passage du courant délivré à l'une desdites électrodes (22, 25) en fonction de celui-ci, et
un moyen (82) destiné à comparer le signal d'asservissement de courant détecté avec un signal de seuil d'excès de courant prédéterminé et produisant le signal de limite d'excès de courant en fonction de leur comparaison.
